# EUROPÄISCHE PATENTANMELDUNG

(11) **EP 0 818 485 A2**
(43) Veröffentlichungstag der Anmeldung: **14.01.1998**
(21) Anmeldenummer: 97110473.2
(22) Anmeldetag: 26.06.1997
(51) Int. Cl.: C08G 18/79, C07D 251/34

(54) **Verfahren zur Herstellung von Isocyanuratgruppen aufweisenden Polyisocyanaten, die nach diesem Verfahren erhältlichen Verbindungen und ihre Verwendung zur Herstellung von Polyisocyanat-Additionsprodukten**

(30) Priorität: 10.07.1996 DE 19627825
(71) Anmelder: BAYER AG, 51368 Leverkusen (DE)
(72) Erfinder: Scholl, Hans-Joachim, Dr., 51061 Köln (DE); Jansen, Bernhard, Dr., 51061 Köln (DE); Meyer, Rolf-Volker, Dr., 51373 Leverkusen (DE)

(57) **Zusammenfassung**

Die Erfindung betrifft ein neues Verfahren zur Herstellung von Isocyanuratgruppen aufweisenden Polyisocyanaten durch teilweise Trimerisierung der Isocyanatgruppen von organischen Polyisocyanaten unter (Mit)verwendung von bestimmten, nachstehend näher beschriebenen Homologen- und Isomerengemischen von cycloaliphatischen Diisocyanaten als Ausgangspolyisocyanat, die nach diesem Verfahren erhältlichen Verbindungen, sowie ihre Verwendung bei der Herstellung von Polyisocyanat-Additionsprodukten.

## Beschreibung

Die Erfindung betrifft ein neues Verfahren zur Herstellung von Isocyanuratgruppen aufweisenden Polyisocyanaten durch teilweise Trimerisierung der Isocyanatgruppen von organischen Polyisocyanaten unter (Mit)verwendung von bestimmten, nachstehend näher beschriebenen Homologen- und Isomerengemischen von cycloaliphatischen Diisocyanaten als Ausgangspolyisocyanat, die nach diesem Verfahren erhältlichen Verbindungen, sowie ihre Verwendung bei der Herstellung von Polyisocyanat-Additionsprodukten.

Verfahren zur Herstellung von Isocyanuratgruppen aufweisenden Polyisocyanaten sind in großer Zahl bekannt geworden (J.H. Saunders und K.C. Frisch, Polyurethanes Chemistry and Technology, Part I, S. 94 ff. (1962). Spezielle Verfahren zur Herstellung cycloaliphatischer und gemischt cycloaliphatischer/aliphatischer Polyisocyanate mit Isocyanuratgruppen sind nochmals zusammenfassend in jüngerer Zeit beschrieben worden (H.J. Laas, R. Halpaap und J. Pedain, J. prakt. Chem. 336 (1994), 185-200). Bei diesen Verfahren werden Trimerisierungskatalysatoren der unterschiedlichsten, in den genannten Literaturstellen beschriebenen Art verwendet.

Vielen Isocyanuratgruppen aufweisenden Polyisocyanaten haften indessen noch eine Reihe verbesserungswürdiger Nachteile an: Verträglichkeitsprobleme im Zuge der Abmischung mit den anderen Komponenten und zu kurze Standzeiten der Abmischungen, insbesondere bei einer Verwendung als Verleimungsmittel.

Es war daher Aufgabe der vorliegenden Erfindung, neue, auf einfachem Wege zugängliche Isocyanuratgruppen aufweisende Polyisocyanate zur Verfügung zu stellen, mit verbesserter Verträglichkeit und verbesserten Standzeiten. Diese Aufgabe konnte mit dem nachstehend näher beschriebenen erfindungsgemäßen Verfahren gelöst werden.

Gegenstand der Erfindung ist ein Verfahren zur Herstellung von Isocyanuratgruppen aufweisenden Polyisocyanaten durch Trimerisierung eines Teils der Isocyanatgruppen von organischen Polyisocyanaten in Gegenwart von Trimerisationskatalysatoren und Abbruch der Trimerisierungsreaktion beim jeweils gewünschten Trimerisierungsgrad, dadurch gekennzeichnet, daß man
a) als zu trimerisierendes Polyisocyanat ein Homologen- und Isomerengemisch von Diisocyanaten der Formel (1) 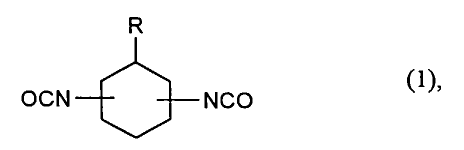 in welcher
   - R: für einen gesättigten, geradkettigen, aliphatischen Kohlenwasserstoffrest mit 8 bis 15 Kohlenstoffatomen steht,
   und gegebenenfalls
b) bis zu 95 NCO-Äquivalent-%, bezogen auf die Summe a) + b), an anderen organischen Polyisocyanaten verwendet.

Gegenstand der Erfindung sind auch die nach diesem Verfahren erhältlichen, Isocyanuratgruppen aufweisenden Polyisocyanate.

Gegenstand der Erfindung ist auch die Verwendung der nach diesem Verfahren erhältlichen, Isocyanuratgruppen aufweisenden Polyisocyanate, gegebenenfalls in von überschüssigen Ausgangspolyisocyanaten befreiter Form und/oder gegebenenfalls in mit Blockierungsmittel für Isocyanatgruppen blockierter Form als Isocyanatkomponente zur Herstellung von Polyisocyanat-Additionsprodukten.

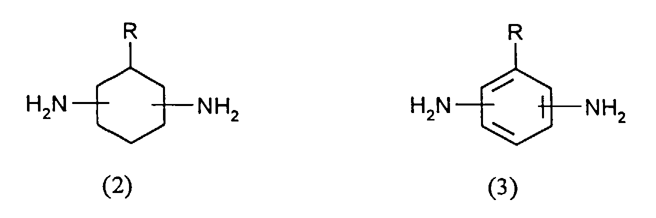

Beim erfindungsgemäßen Verfahren werden als erfindungswesentliche Ausgangskomponente a) Diisocyanate der bereits genannten Formel (1) eingesetzt. Die Herstellung erfolgt durch Kernhydrierung zu Verbindungen der Formel (2) und anschließende Phosgenierung der so erhaltenen Aminogruppen, ausgehend von als Homologen- und Isomerengemisch vorliegenden aromatischen Diaminen der Formel (3), herstellbar gemäß EP 0 058 335.

Die erfindungswesentliche Komponente a) wird beim erfindungsgemäßen Verfahren entweder als alleinige Ausgangskomponente oder zusammen mit bis zu 95 NCO-Äquivalent-%, bezogen auf alle Isocyanatgruppen der Ausgangspolyisocyanate, an anderen Polyisocyanaten b) eingesetzt.

Bei diesen gegebenenfalls mitzuverwendenden Polyisocyanaten b) handelt es sich beispielsweise um
b1) aliphatische oder cycloaliphatische Polyisocyanate eines über 139, vorzugsweise bei 140 bis 250 liegenden Molekulargewichts, wie z.B. Tetramethylendiisocyanat, Hexamethylendiisocyanat, Dodecanmethylendiisocyanat, Cyclobutan-1,3-diisocyanat, Cyclohexan-1,3- und -1,4-diisocyanat, 1-Isocyanato-3,3,5-trimethyl-5-isocyanatomethyl-cyclohexan (IPDI), Perhydro-2,4- und/oder -2,6-diisocyanatotoluol oder Perhydro-2,4'- und/oder -4,4'-diisocyanatodiphenylmethan, 1-Isocyanato-1-methyl-4(3)-isocyanatomethylcyclohexan (IMCI);
   oder um
b2) aromatische Polyisocyanate eines über 173, vorzugsweise bei 174 bis 250 liegenden Molekulargewichts, wie z.B. 2,4- oder 2,6-Toluylendiisocyanat, 2,4'-Diphenylmethandiisocyanat, 4,4'-Diphenylmethandiisocyanat, Naphthylen-1,5-diisocyanat, 4,4',4''-Triisocyanatotriphenylmethan, 2,4,6-Triisocyanatotoluol oder Polyphenyl-polymethylen-polyisocyanate, wie sie durch Anilin-Formaldehyd-Kondensation und anschließende Phosgenierung hergestellt werden ("Roh-MDI");
b3) einen NCO-Gehalt von ca. 1 bis ca. 11 Gew.-% aufweisende NCO-Präpolymere auf Basis von (i) den erfindungswesentlichen Diisocyanaten a) und/oder den unter b1) und b2) genannten Polyisocyanaten und (ii) den aus der Polyurethanchemie insbesondere für 2-Komponenten-Polyurethanlacke bekannten Polyhydroxylverbindungen des Molekulargewichtsbereiches 62 bis 5.000, vorzugsweise 200 bis 2.000 der in US-PS 4 289 813, Kolonne 3, Zeile 47 - Kolonne 4, Zeile 12 beispielhaft offenbarten Art.

Beliebige Gemische der unter b1) bis b3) beispielhaft genannten Polyisocyanate können ebenfalls beim erfindungsgemäßen Verfahren als Komponente b) eingesetzt werden. Vorzugsweise werden die unter b1) beispielhaft genannten aliphatischen und cycloaliphatischen Diisocyanate eingesetzt.

Als Trimerisierungskatalysatoren kommen alle bekannten Trimerisierungskatalysatoren des Standes der Technik in Betracht, wie sie beispielsweise in den obengenannten Veröffentlichungen beispielhaft genannt sind. Bevorzugte Trimerisierungskatalysatoren sind die beispielsweise in DE-OS 2 551 634 beschriebenen Mannich-Basen.

Besonders bevorzugt sind die in EP 330 966 und EP 355 479 beschriebenen quartären Ammoniumhydroxide und Ammoniumfluoride.

Die Beendigung der erfindungsgemäßen Trimerisierungsreaktionen erfolgt entweder, bei Verwendung von thermisch labilen Trimerisierungskatalysatoren, thermisch oder vorzugsweise durch Zugabe von Katalysatorengiften der beispielsweise in den zuletzt genannten Veröffentlichungen genannten Art. Bei Ammoniumfluorid-Katalyse werden als Katalysatorgifte vorzugsweise silylierte Säsuren gemäß EP 508 216 zugegeben.

Das erfindungsgemäße Verfahren kann sowohl lösungsmittelfrei, als auch in Anwesenheit von inerten Lösungs- und Verdünnungsmitteln durchgeführt werden. Als inerte Lösungsmittel können unpolare Verdünungsmittel wie Toluol, Xylol, höhere Aromaten, Leichtbenzin, Testbenzin und C₁₂-C₂₀-Alkylsulfonsäureester, aber auch inerte polare Lösungsmittel wie Ester und Ketone bzw. Gemische derartiger Lösungsmittel eingesetzt werden.

Bevorzugt setzt man Gemische von aromatischen Kohlenwasserstoffen und Estern ein, z.B. ein Gemisch von Butylacetat und Xylol.

Die erfindungsgemäße Trimerisierungsreaktion wird im allgemeinen im Temperaturbereich zwischen 10 bis 200°C, vorzugsweise 20 bis 80°C durchgeführt. Die optimale Reaktionstemperatur richtet sich nach der Art der eingesetzten Ausgangspolyisocyanaten und der Art der eingesetzten Trimerisierungskatalysatoren und kann in einem einfachen Vorversuch ermittelt werden.

Die erfindungsgemäße Trimerisierungsreaktion wird im allgemeinen bei Erreichen eines Trimerisierungsgrads (Trimerisierungsgrad = Prozentsatz der trimerisierten Isocyanatgruppen, bezogen auf Gesamtmenge der im Ausgangspolyisocyanat vorliegenden Isocyanatgruppen) von 10 bis 70 % abgebrochen. Der Verlauf der Reaktion kann z.B. durch fortlaufende Bestimmung des Brechungsindex verfolgt werden.

Bei der lösungsmittelfreien Durchführung des erfindungsgemäßen Verfahrens, gegebenenfalls unter anschließender Entfernung von überschüssigem Ausgangsisocyanat, beispielsweise im Dünnschichtverdampfer, liegt der Trimerisierungsgrad im allgemeinen zwischen 10 und 50 %. Bei der Durchführung des erfindungsgemäßen Verfahrens in Gegenwart von Lösungsmitteln ohne anschließende Entfernung von nicht-umgesetztem Ausgangsisocyanat liegt der Trimerisierungsgrad im allgemeinen zwischen 50 und 70 %.

Dabei ist es ein besonderer Vorteil des erfindungsgemäßen Verfahrens, daß bei einem Trimerisierungsgrad von ca. 50 bis 70 % der Gehalt an monomeren Isocyanaten überraschenderweise sehr niedrig ist und eine anschließende Entfernung von monomeren Isocyanaten durch Extraktion oder Destillation nicht notwendig ist.

Der Abbruch der Trimerisierungsreaktion erfolgt, wie bereits dargelegt, durch thermische oder chemische Desaktivierung des Katalysators.

Vorzugsweise werden beim erfindungsgemäßen Verfahren im Falle der Mitverwendung von Ausgangskomponenten b) vorab hergestellte Gemische der Komponenten a) und b) eingesetzt. Gemäß einer besonderen Ausführungsform des erfindungsgemäßen Verfahrens ist es jedoch auch möglich, zunächst in einem ersten Reaktionsschritt einen Teil, d.h. bis maximal 5 %, der Isocyanatgruppen der Komponente a) oder der Komponente b) zu trimerisieren, anschließend die nicht-trimerisierte Komponente b) oder a) dem Reaktionsgemisch hinzuzufügen und die Trimerisierungsreaktion zum Abschluß zu bringen.

In einem solchen Falle muß jedoch darauf geachtet werden, daß die Beendigung der Trimerisierungsreaktion frühestens dann erfolgt, wenn nach erfolgter Zugabe der nicht-trimerisierten Ausgangskomponente weitere 5 %, vorzugsweise weitere 10 % aller ursprünglich in den Ausgangspolyisocyanaten vorliegender Isocyanatgruppen trimerisiert sind. So ist es beispielsweise möglich von 100 NCO-Äquivalenten der Komponente a) oder b) max. 30 NCO-Äquivalente zu trimerisieren, dem Reaktionsgemisch dann 100 NCO-Äquivalente der Komponente b) oder a) einzuverleiben, die Trimerisierungsreaktion fortzusetzen und im Falle der Trimerisierung von 30 NCO-Äquivalenten der ersten Ausgangskomponente frühestens nach der Trimerisierung von insgesamt 25 NCO-Äquivalenten abzubrechen.

Die erfindungsgemäßen Verfahrensprodukte können insbesondere im Falle der lösungsmittelfreien Durchführung des erfindungsgemäßen Verfahrens in an sich bekannter Weise, beispielsweise durch Dünnschichtdestillation oder Extraktion von überschüssigen, nicht-umgesetzten Ausgangsisocyanten befreit werden, so daß Isocyanuratgruppen aufweisende Polyisocyanate mit einem Gehalt an monomeren Ausgangsisocyanaten unter 3 Gew.-%, vorzugsweise unter 0,7 Gew.-%, bezogen auf die Polyisocyanat-Ausgangskomponente b), erhältlich sind. Die Senkung des Restmonomerengehalts der erfindungswesentlichen Ausgangskomponenten a) ist aufgrund ihres niedrigen Dampfdrucks nicht zwingend, so daß auch Gehalte über 3 Gew.-% akzeptabel sind.

Die Entfernung von überschüssigen Ausgangsisocyanaten erfolgt vorzugsweise, wenn eine Anwendung der Verfahrensprodukte für Polyurethanlacke vorgesehen ist. Vor ihrer Verwendung als Isocyanat-Komponente in Zweikomponenten-Polyurethanlacken können die erfindungsgemäßen Verfahrensprodukte noch modifiziert werden, beispielsweise durch Einführung von Urethan-, Harnstoff-, Biuret- oder Allophanatgruppen.

Selbstverständlich ist eine Verwendung der erfindungsgemäßen Verfahrensprodukte auch ohne Entfernung von überschüssigen Ausgangsisocyanaten möglich, beispielsweise zur Herstellung von Leimungsmitteln.

Die erfindungsgemäßen Verfahrensprodukte können selbstverständlich in an sich bekannter Weise mit geeigneten Blockierungsmitteln für Isocyanatgruppen wie z.B. Phenol, ε-Caprolactam, Maliensäurediethylester oder Acetessigsäureethylester blockiert werden.

Die erfindungsgemäßen Verfahrensprodukte bzw. ihre durch die genannte Blockierungsreaktion erhaltenen Derivate stellen wertvolle Ausgangsmaterialien zur Herstellung von Polyurethankunststoffen nach dem Isocyanat-Polyadditionsverfahren dar. Sie eignen sich insbesondere für Verleimungen und als Isocyanatkomponente in Zweikomponentenpolyurethanlacken.

Die folgenden Beispiele erläutern die Erfindung. Alle Prozentangaben betreffen, soweit nicht anderslautend ausgeführt, Gewichtsprozente. Alle Angaben in "Teilen" betreffen Gewichtsteile. Alle Angaben in "NCO-Äquivalent-%" beziehen sich auf die Gesamtmenge der in den Beispielen eingesetzten Ausgangsisocyanate.

### Herstellung eines Diisocyanats der Formel (1)

### Beispiel 1

### Kernhydrierung zur Herstellung des Amins der Formel (2)

In diesem Beispiel wird ein als Homologen- und Isomerengemisch vorliegendes aromatisches Diamin (3) gemäß EP 0 058 335 eingesetzt, deren Alkylketten eine Länge von 10 bis 13 C-Atomen bei einer mittleren Kettenlänge von etwa 12 C-Atomen aufweisen.

In einem 3 l-Rührautoklaven werden 776 g Diamin, 763 g tert.-Butanol und 7,7 g Rutheniumoxidhydrat vorgelegt. Der Autoklav wird dreimal mit Stickstoff gespült. Anschließend werden 138 bar Wasserstoff aufgedrückt, der Ansatz unter Rühren auf 180°C erhitzt und das eingesetzte aromatische Diamin im Druckbereich von 270 bis 259 bar kernhydriert. Nach einer Reaktionszeit von 5 Stunden ist die Wasserstoffaufnahme beendet. Nach Entspannen des Autoklaven und Abtrennen des Katalysators wird das Filtrat am Rotationsverdampfer eingeengt und das Rohamin nachfolgend unter vermindertem Druck destilliert. Auf diese Weise können 739 g Diamingemisch als bei 130 bis 152°C/0,2 mbar siedende Fraktion erhalten werden (Ausbeute: 95 %).

### Beispiel 2

In diesem Beispiel wird das als Homologen- und Isomerengemisch vorliegende Diamin gemäß Beispiel 1 eingesetzt, dessen Alkylketten eine Länge von 10 bis 13 C-Atomen bei einer mittleren Kettenlänge von etwa 12 C-Atomen aufweisen.

In einem 4 l Vierhalskolben mit Rührer, Thermometer, Gaseinleitungsrohr und Rückflußkühler versehen, werden 2 l trockenes Chlorbenzol vorgelegt. Unter Rühren und Kühlung (-10°C) werden 500 g Phosgen einkondensiert, anschließend läßt man unter Kühlung bei -10 bis -5°C 370 g Diamin, in 300 g Chlorbenzol gelöst, zutropfen. Unter weiterem Einleiten von Phosgen erwärmt man langsam bis auf Rückflußtemperaturen. Nach Ende der Chlorwasserstoffentwicklung wird das überschüssige Phosgen mit Stickstoff ausgeblasen und die Lösung im Vakuum eingedampft. Man erhält 345 g Rohisocyanatgemisch mit einem NCO-Gehalt von 19,1 % (Theorie: 25,1 %), das nach IR-spektroskopischen Untersuchungen geringe Biuret-Anteile enthält.

300 g des Rohprodukts werden unter vermindertem Druck destilliert. Bei einem Druck von 0,2 mbar destillieren innerhalb des Temperaturbereichs von 150 bis 175°C 225 g eines praktisch farblosen Gemischs von Diisocyanaten der Formel (1) 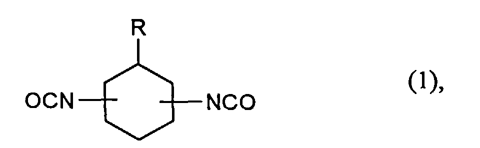 in der
- R: obige Bedeutung hat.

### Herstellung der Trimerisierungs-Katalysatoren A und B gemäß EP 355 479

### Katalysator A

Zu 4,8 g Kaliumfluorid in 320 g 1-Butanol aufgeschlämmt, werden unter Rühren bei Raumtemperatur 32,4 g Tricaprylmethylammoniumchlorid (Aliquat 336 der Fa. Fluka), in 60 g 1-Butanol gelöst, innerhalb von 20 Minuten zugetropft. Man rührt 60 Minuten nach, filtriert von unlöslichen Bestandteilen ab und erhält eine Katalysatorlösung mit den Daten: F⁻: 0,16 mmol/g.

### Katalysator B

Zu 4,8 g Kaliumfluorid in 640 g 1-Butanol aufgeschlämmt, werden unter Rühren bei Raumtemperatur 32,4 g Tricaprylmethylammoniumchlorid (Aliquat 336 der Fa. Fluka), in 120 g 1-Butanol gelöst, innerhalb von 20 Minuten zugetropft. Man rührt 30 Minuten nach, filtriert von unlöslichen Bestandteilen ab und erhält eine Katalysatorlösung mit den Daten: F⁻: 0,09 mmol/g.

### Trimerisierungsbeispiele:

In den nachfolgenden Trimerisierungsbeispielen 3 bis 6 wird als "Diisocyanat 1" ein Diisocyanatgemisch gemäß Beispiel 2 eingesetzt.

### Beispiel 3 (Teiltrimerisierung des Diisocyanats 1)

322 g (0,96 Mol) Diisocyanat 1 werden unter Stickstoffatmosphäre auf 60°C erwärmt. Anschließend fügt man 1,5 g Katalysator A hinzu und prüft den Reaktionsfortgang über zunehmende Brechungsindices. Nach 30 Minuten bei 60 bis 70°C ist der Brechungsindex (25°C) von 1,4775 auf 1,4879 gestiegen, gleichbedeutend mit einem NCO-Abfall von 25,0 auf 19,3 %. Die Reaktion wird mit 60 mg Trimethylsilyl-trifluormethansulfonat (TMS triflat), in 2 g Diisocyanat 1 gelöst, gestoppt und das Reaktionsgemisch 30 min bei 80°C nachgerührt. Man erhält als Klare, farblose Lösung ein Isocyanuratgruppen aufweisendes Polyisocyanatgemisch mit den Daten:
NCO-Gehalt: 19,3 %
Viskosität (25°C): 327 mPa.s

### Beispiel 4 (Teiltrimerisierung des Diisocyanats 1)

278 g (0,83 Mol) Diisocyanat 1 werden unter Stickstoffatmosphäre auf 60°C erwärmt. Anschließend fügt man 0,5 g Katalysator A hinzu und prüft den Reaktionsfortgang über zunehmende Brechungsindices. Nach 2 Stunden bei 60 bis 65°C ist der Brechinngsindex (25°C) von 1,4772 auf 1,4826 gestiegen, gleichbedeutend mit einem NCO-Abfall von 25,0 auf 22,0 %. Die Reaktion wird mit 0,3 g einer Lösung von 60 mg Trimethylsilyl-trifluormethansulfonat (TMS triflat), in 1 g Diisocyanat 1 gelöst, gestoppt und das Reaktionsgemisch 30 min bei 80°C nachgerührt. Man erhält als klare, farblose Lösung ein Isocyanuratgruppen aufweisendes Polyisocyanatgemisch mit den Daten:
NCO-Gehalt: 22,0 %
Viskosität (25°C): 100 mPa.s

### Beispiel 5 (Cotrimerisierung eines Gemischs von Diisocyanat 1 und Isophorondiisocyanat (IPDI))

Ein Gemisch von 160 g (0,5 mol) Diisocyanat 1 und 444 g (2 mol) IPDI wird auf 60°C erwärmt und mit 6,1 g Katalysator A versetzt. Die exotherm einsetzende Reaktion wird durch Kühlung auf 70°C gehalten und nach 30 min mit 3 g einer Lösung von 0,7 g Trimethylsilyltrifluormethansulfonat (TMS triflat), in 9,3 g IPDI gelöst, gestoppt. Man läßt 30 min bei 80°C nachrühren und erhält ein Isocyanuratgruppen enthaltendes Gemisch mit einem NCO-Gehalt von 26,9 %. Nach Abtrennen der nicht umgesetzten Diisocyanate durch Dünnschichtdestillation erhält man 240 g (Ausbeute: 40 %) eines klaren, hellgelb gefärbten Harzes mit einem NCO-Wert von 13,9 %. Nach gaschromatographischen Untersuchungen des eingesetzten Gemisches im Vergleich zum gedünnschichteten Destillat sind beide Diisocyanate zu gleichen Gewichtsanteilen, bezogen auf die jeweiligen Einsatzmengen, eingebaut worden.

### Beispiel 6 (Cotrimerisierung eines Gemischs von Diisocyanat 1 und Hexamethylendiisocyanat (HDI))

Ein Gemisch von 165 g (0,5 mol) Diisocyanat 1 und 252 g (1,5 mol) HDI wird auf 55°C erwärmt und mit 1,2 g Katalysator B versetzt. Die leicht exotherme Reaktion wird auf 60°C gehalten und ihr Fortgang innerhalb der nächsten 2 Stunden durch weiteren Zusatz von 1,4 g Katalysator B erzwungen. Nach einer weiteren Stunde bei 60°C wird mit 0,9 g einer Lösung von 0,7 g Trimethylsilyltrifluormethansulfonat (TMS triflat), in 9,3 g HDI gelöst, abgestoppt. Man läßt 30 min bei 80°C nachrühren und erhält ein Isocyanuratgruppen enthaltendes, farbloses Gemisch mit einem NCO-Gehalt von 35,2 %. Nach Abtrennen der nicht umgesetzten Diisocyanate durch Dünnschichtdestillation erhält man 86 g (Ausbeute: 21 %) einer klaren, fast farblosen Flüssigkeit mit einem NCO-Wert von 18,6 % und einer Viskosität (25°C) von 4300 mPa.s. Nach gaschromatographischen Untersuchungen des eingesetzten Gemisches im Vergleich zum gedünnschichteten Destillat sind ca. 52 g HDI und ca. 34 g Diisocyanat 1 eintrimerisiert worden.

### Verwendungsbeispiele

### Anwendungsbeispiel 1 (Zubereitung der Leimflotte für die Masseleimung)

2,5 g kationische Stärke werden bei 50°C in 187,5 g Wasser gelöst. In dieser klaren Lösung werden mit einem ULTRA-TURRAX (Fa. IKA) 10 g des antrimerisierten Isocyanates aus Beispiel 4 dispergiert. Nach ca. 15 Min. ergibt sich eine völlig homogene milchige Dispersion.

### Anwendungsbeispiel 2 (Blattbildung und Leimungsprüfung mit der Isocyanatdispersion aus Anwendungsbeispiel 1)

Auf einem Blattbildner der Bauart Rapid-Köthen werden bei pH 7 Papiere mit einem Flächengewicht von 80 g/m² hergestellt. Der Zellstoff besteht aus 80 % Nadelholzzellstoff und 20 % Laubholzzellstoff mit einem Mahlgrad von 35° SR; als Füllstoff werden 30 % gefälltes CaCO₃ eingesetzt. An Leimungsmittel werden 0,3 bzw. 0,5 % Wirksubstanz, bezogen auf Zellstoff und Füllstoff zusammen, eingesetzt. Zur Verbesserung der Retention wird noch ein Retentionsmittel (Retaminol CO1 der Fa. Bayer AG) mit 0,2 % der Handelsware bezogen auf Zellstoff und Füllstoff zugegeben. Die noch feuchten Papiere werden 10 Minuten im Trockenschrank bei 90°C nachkondensiert.

Die Leimungswirkung wird durch den Cobb-Test geprüft. Hierbei wird nach DIN 53 132 die einseitige Wasseraufnahme eines Papieres innerhalb von 60 Sekunden gravimetrisch ermittelt.

Der gefundene Wert ist eine Maßzahl für den Leimungsgrad; die Werte sind in der folgenden Tabelle zusammengefaßt.

| Leimungsmittel | Einsatzmenge (% Wirksubstanz) | Cobb-Wert (g/m²) |
|---|---|---|
| Aquapel 2B* | 0,3 | 23,0 |
| Aquapel 2B* | 0,5 | 19,3 |
| Leimungsflotte aus Anwendungsbeispiel 1 | 0,3 | 16,9 |
| | 0,5 | 17,0 |

| | | |
|---|---|---|
| * enthält 12 % Wirksubstanz (Produkt der Fa. Hercules, Vergleich) | | |

### Anwendungsbeispiel 3 (Herstellung eines wasserdispergierbaren Isocyanates)

74 g des antrimerisierten Isocyanates aus Beispiel 4 werden mit 25 g eines auf Ethylenglykolmonomethylether mit einem mittleren Molekulargewicht von 350 gestarteten Polyether und 1 g Dimethylethanolamin bei 60°C umgesetzt. Es wird gerührt bis der Isocyanatgehalt 10,5 % beträgt. Das Isocyanat ist in Wasser durch einfaches Einrühren mittels eines Glasstabes im Becherglas sehr gut dispergierbar.

### Anwendungsbeispiel 4 (Herstellung eines wasserdispergierbaren Isocyanates)

74 g des Mischtrimerisates aus Beispiel 6 werden wie oben beschrieben, mit 25 g des obengenannten Polyethers und 1 g Dimethylethanolamin umgesetzt. Der Isocyanatgehalt beträgt 9,3 %; das Isocyanat ist unter den obengenannten Bedingungen sehr gut dispergierbar.

### Anwendungsbeispiel 5 (Oberflächenleimung von Papier und Prüfung des Leimungsgrades)

Kreidehaltiges Papier mit einem Flächengewicht von 80 g/m² wird auf einer Labor-Leimpresse der Fa. Mathis, Zürich, Schweiz, Typ HF mit einer wäßrigen Dispersion der Isocyanate aus den Beispielen 3 und 4 behandelt. Die Leimungsflotten enthielten außer den in der folgenden Tabelle genannten Gehalten der Leimungsmittel noch 5 % Stärke (Perfektamyl der Fa. AVEBE, Niederlande).

Die dergestalt ausgerüsteten Papiere werden durch Abpressen mit Filz entwässert und dann 10 Minuten bei 90°C im Trockenschrank getrocknet. Die Leimungswirkung wird, wie in Anwendungsbeispiel 2 beschrieben, durch den Cobb-Test geprüft.

Die gefundenen Werte sind in der folgenden Tabelle zusammengefaßt:

| Leimungsmittel | Einsatzkonzentration (% Wirksubstanz) | Cobb-Test (g/m²) |
|---|---|---|
| Aquapel 2B* | 0,15 | 22,4 |
| | 0,30 | 20,4 |
| | 0,45 | 18,7 |
| Anw.-Beispiel 3 | 0,15 | 25,2 |
| | 0,30 | 25,4 |
| | 0,45 | 25,2 |
| Anw.-Beispiel 4 | 0,15 | 24,6 |
| | 0,30 | 32,8 |
| | 0,45 | 23,1 |

| | | |
|---|---|---|
| * enthält 12 % Wirksubstanz (Produkt der Fa. Hercules, Vergleich) | | |

## Patentansprüche

1. Verfahren zur Herstellung von Isocyanurat-Gruppen aufweisenden Polyisocyanaten durch Trimerisierung eines Teils der Isocyanatgruppen von organischen Polyisocyanaten in Gegenwart von Trimerisierungskatalysatoren und Abbruch der Trimerisierungsreaktion beim jeweils gewünschten Trimerisierungsgrad, dadurch gekennzeichnet, daß man
a) als zu trimerisierendes Polyisocyanat ein Homologen- und Isomerengemisch von Diisocyanaten der Formel (1) 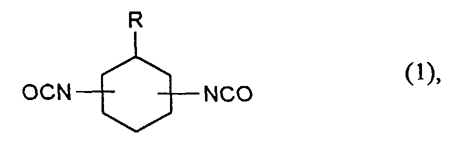 in welcher
R für einen gesättigten, geradkettigen, aliphatischen Kohlenwasserstoffrest mit 8 bis 15 Kohlenstoffatomen steht,
und gegebenenfalls
b) bis zu 95 NCO-Äquivalent-%, bezogen auf die Summe a) + b), an anderen organischen Polyisocyanaten verwendet.

2. Ausführungsform des Verfahrens gemäß Anspruch 1, dadurch gekennzeichnet, daß man in einem ersten Reaktionsschritt bis maximal 30 % der Isocyanatgruppen der Ausgangspolyisocyanate a) oder b) trimerisiert, anschließend die zweite Ausgangskomponente b) oder a) dem Reaktionsgemisch hinzufügt und die Reaktion zu Ende führt, wobei der Abbruch der Trimerisierungsreaktion frühestens dann erfolgt, wenn mindestens weitere 5 % der Isocyanatgruppen, bezogen auf alle Isocyanatgruppen der untrimerisierten Ausgangspolyisocyanate, trimerisiert sind.

3. Verfahren gemäß Anspruch 1 und 2, dadurch gekennzeichnet, daß man als Ausgangspolyisocyanate a) als Homologen- und Isomerengemisch vorliegende Diisocyanate der in Anspruch 1 genannten Formel verwendet, für welche R für einen gesättigten, geradkettigen, aliphatischen Kohlenwasserstoffrest mit 8 bis 15 Kohlenstoffatomen steht.

4. Verfahren gemäß Anspruch 1 bis 3, dadurch gekennzeichnet, daß man als Ausgangspolyisocyanat b) Hexamethylendiisocyanat oder 1-Isocyanato-3,3,5-trimethyl-5-isocyanatomethyl-cyclohexan verwendet.

5. Gemäß Anspruch 1 bis 4 erhältliche, Isocyanurat-Gruppen aufweisende Polyisocyanate.

6. Verwendung der gemäß Anspruch 1 bis 4 erhältlichen, Isocyanurat-Gruppen aufweisenden Polyisocyanate, gegebenenfalls in von überschüssigen Ausgangspolyisocyanaten befreiter Form und/oder gegebenenfalls in mit Blockierungsmitteln für Isocyanat-Gruppen blockierter Form als Isocyanatkomponente zur Herstellung von Polyurethanen nach dem Isocyanat-Polyadditions-Verfahren.
